(19) Europäisches Patentamt
European Patent Office
Office européen des brevets

(11) Veröffentlichungsnummer: **0 252 505 B1**

(12) **EUROPÄISCHE PATENTSCHRIFT**

(45) Veröffentlichungstag der Patentschrift: **16.09.92**

(51) Int. Cl.[5]: **C07F 9/38**, C07F 9/40, A61K 31/66

(21) Anmeldenummer: **87109900.8**

(22) Anmeldetag: **09.07.87**

(54) **1-Hydroxy-3-(N-methyl-N-propylamino)propan-1,1-diphosphonsäure; Verfahren zu dessen Herstellung und diese Verbindung enthaltende Arzneimittel.**

(30) Priorität: **11.07.86 DE 3623397**

(43) Veröffentlichungstag der Anmeldung:
**13.01.88 Patentblatt 88/02**

(45) Bekanntmachung des Hinweises auf die Patenterteilung:
**16.09.92 Patentblatt 92/38**

(84) Benannte Vertragsstaaten:
**AT BE CH DE ES FR GB GR IT LI LU NL SE**

(56) Entgegenhaltungen:
**DE-A- 2 534 391**
**SU-A- 1 002 300**

(73) Patentinhaber: **BOEHRINGER MANNHEIM GMBH**
**Sandhofer Strasse 116**
**W-6800 Mannheim 31(DE)**

(72) Erfinder: **Gall, Rudi, Dr. phil.**
**Ulmenstrasse 1**
**W-6945 Hirschberg(DE)**
Erfinder: **Bosies, Elmar, Dr. rer. nat.**
**Delpstrasse 11**
**W-6940 Weinheim(DE)**

**EP 0 252 505 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft 1-Hydroxy-3-(N-methyl-N-propylamino)propan-1,1-diphosphonsäure, Verfahren zu dessen Herstellung sowie Arzneimittel, die diese Substanz enthalten.

In der DE-OS 18 13 659 sind Diphosphonsäurederivate beschrieben, von denen die 1-Hydroxy-ethan-1,1-diphosphonsäure als Mittel zur Behandlung von Morbus Paget Bedeutung erlangt hat.

In den Patentschriften DE-OS 29 43 498, DE-OS 27 02 631, EP 96-931-A sowie in Z. Anorg. Allg. Chem. 457, 214 (1979) sind 1-Hydroxy-3-(N,N-dialkylamino)propan-1,1 diphosphonsäuren beschrieben, die als gute Calciumkomplexbildner bekannt sind, die sich aber auch zur Behandlung der erhöhten Knochenresorption einsetzen lassen. In der DE-OS 25 34 391 ist die 1-Hydroxy-3-(N-methyl-N-propylamino)propan-1, 1-disphosphonsäure von der allgemeinen Formel mit umfaßt, aber nicht als Beispiel beschrieben oder als bevorzugte Verbindung genannt.

Überraschenderweise wurde jetzt gefunden, daß diese am Stickstoff unsymmetrisch dialkylierte Verbindung gegenüber den in dieser Patentschrift beschriebenen Verbindungen neben einer ebenfalls sehr guten Calciumkomplexierung eine deutlich bessere Wirkung bei Knochenstoffwechselerkrankungen bei guter Verträglichkeit zeigt.

Die erfindungsgemäße Verbindung läßt sich vor allem sehr gut dort einsetzen, wo der Knochenauf- und -abbau gestört ist, d.h. sie ist geeignet zur Behandlung von Erkrankungen des Skelettsystems wie z.B. Osteoporose, Morbus Paget, Morbus Bechterew u.a..

Aufgund dieser Eigenschaften findet sie aber auch Verwendung in der Therapie von Knochenmetastasen, der Urolithiasis und zur Verhinderung heterotoper Ossifikationen. Durch ihre Beeinflussung des Calciumstoffwechsels bildet sie weiterhin eine Grundlage für die Behandlung der rheumatoiden Arthritis, der Osteoarthritis und der degenerativen Arthrose.

Gegenstand der vorliegenden Erfindung ist die 1-Hydroxy-3-(N-methyl-N-propylamino)propan-1,1-diphosphonsäure der Formel I

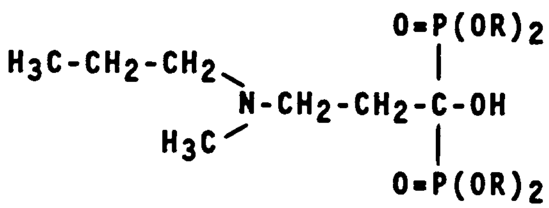

(I)

in der R Wasserstoff oder eine $C_1$ - $C_4$-Alkylgruppe bedeutet, und pharmakologisch unbedenkliche Salze. Bei den erfindungsgemäßen Di- bzw. Tetraestern handelt es sich vorzugsweise um die Methyl-, Ethyl- oder Isobutylester.

Die Verbindung der Formel I wird nach an sich bekannten Verfahren hergestellt, vorzugsweise indem man

a) eine Carbonsäure der Formel II

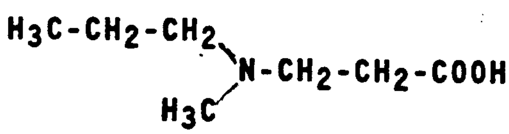

(II),

mit einem Gemisch aus phosphoriger Säure oder Phosphorsäure und einem Phosphorhalogenid oder Phosphorylhalogenid umsetzt und anschließend zur freien Diphosphonsäure verseift,
oder

b) ein Carbonsäurechlorid der Formel III

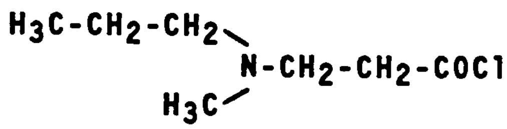 (III),

mit einem Trialkylphosphit der allgemeinen Formel IV

P(OR')$_3$ (IV),

in der R' für Alkylreste mit 1-4 Kohlenstoffatomen, vorzugsweise Methyl, Ethyl und Isobutyl steht, zu einem Acylphosphonat der Formel V

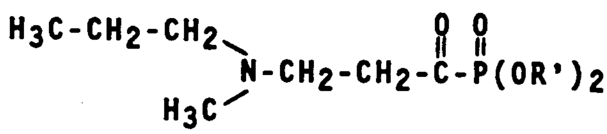 (V),

in der R' die oben angegebene Bedeutung hat,
umsetzt, anschließend mit einem Dialkylphosphit der allgemeinen Formel VI

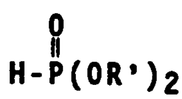 (VI),

in der R' die oben angegebene Bedeutung hat, zu einem Diphosphonat der Formel VII

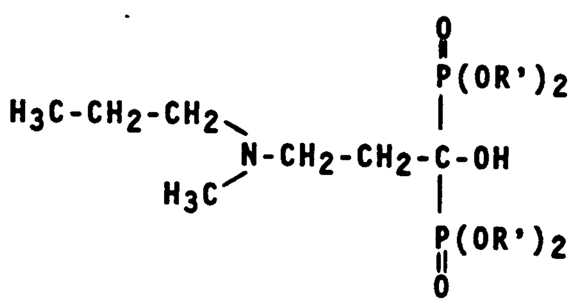 (VII),

in der R' die oben angegebene Bedeutung hat,
reagieren läßt und gegebenenfalls die entstandenen Tetraester zu Diestern oder zur freien 1-Hydroxy-3-(N-methyl-N-propylamino)propan-1,1-diphosphonsäure der Formel I verseift,
oder

c) eine Verbindung der allgemeinen Formel VIII

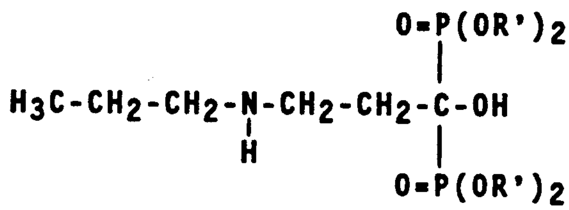 (VIII),

in der R' die oben angegebene Bedeutung hat, methyliert und gegebenenfalls die entstandenen Tetraester zu Diestern oder zur freien 1-Hydroxy-3-(N-methyl-N-propylamino)propan-1,1-diphosphonsäure der Formel I verseift und die so hergestellten Verbindungen gegebenenfalls in ihre pharmakologisch verträglichen Salze überführt.

Die bei Verfahren a) eingesetzte Carbonsäure der Formel II wird mit 1 - 5, vorzugsweise 2 - 3 mol phosphoriger Säure oder Phosphorsäure und 1 - 5, vorzugsweise 2 - 3 mol Phosphortrihalogenid oder Phosphorylhalogenid bei Temperaturen von 80 - 130 ° C, vorzugsweise 80 - 100 ° C umgesetzt. Man kann die Reaktion auch in Gegenwart von Verdünnungsmitteln wie Halogenkohlenwasserstoffen, insbesondere Chlorbenzol, Tetrachlorethan oder auch Dioxan durchführen. Die anschließende Hydrolyse erfolgt durch Kochen mit Wasser, zweckmäßigerweise jedoch mit halbkonzentrierter Salz- oder Bromwasserstoffsäure.

Als Phosphortrihalogenid kommen in dem oben genannten Verfahren beispielsweise Phosphortrichlorid oder Phosphortribromid in Frage, als Phosphorylhalogenid eignet sich Phosphoroxychlorid.

Bei Verfahren b) läßt man das Säurechlorid der Formel III mit dem Trialkylphosphit der allgemeinen Formel IV bei Temperaturen zwischen 0 und 60 ° C, vorzugsweise bei 20 - 40 ° C zur Reaktion kommen. Man kann ohne Lösungsmittel oder auch in Gegenwart von inerten Lösungsmitteln wie Diethylether, Tetrahydrofuran, Dioxan oder auch halogenierten Kohlenwasserstoffen, wie z.B. Methylenchlorid arbeiten. Das als Zwischenprodukt entstehende Acylphosphonat der allgemeinen Formel V kann isoliert oder direkt weiter umgesetzt werden. Die anschließende Reaktion führt man in Gegenwart einer schwachen Base, vorzugsweise einem sec. Amin wie z.B. Dibutylamin bei einer Temperatur von 0 - 60 ° C, vorzugsweise bei 10 - 30 ° C durch.

Bei der reduktiven Alkylierung (Verfahren c)) behandelt man ein Gemisch aus dem sekundärem Amin der allgemeinen Formel VIII und Formaldehyd oder dessen Acetal in Gegenwart eines Hydrierungskatalysators, wie Palladium auf Kohle, oder Nickel, mit Wasserstoff unter Atmosphären-oder erhöhtem Druck oder man setzt als Reduktionsmittel Ameisensäure zu.

Schließlich läßt sich die Methylierung des sekundären Amins der allgemeinen Formel VIII besonders vorteilhaft nach dem Phasentransferverfahren mit Dimethylsulfat durchführen.

Die bei Verfahren b) und c) gegebenenfalls anfallenden Tetraalkylester können zu Diestern oder zur freien 1-Hydroxy-3-(N-methyl-N-propylamino)propan-1,1-diphosphonsäure verseift werden. Die Verseifung zu Diestern geschieht in der Regel dadurch, daß man den Tetraalkylester mit einem Alkalihalogenid, vorzugsweise Natriumjodid in einem geeigneten Lösungsmittel wie z.B. Aceton bei Zimmertemperatur behandelt.

Hierbei entsteht das symmetrische Diester/Dinatriumsalz, das gegebenenfalls durch einen sauren Ionenaustauscher in die Diester/Disäure umgewandelt werden kann. Die Verseifung zur freien Diphosphonsäure geschieht in der Regel durch Kochen mit Salz- oder Bromwasserstoffsäure. Man kann jedoch auch eine Spaltung mit Trimethylsilylhalogenid, vorzugsweise dem Bromid oder Jodid vornehmen. Die freie Diphosphonsäure kann umgekehrt durch Kochen mit Orthoameisensäurealkylestern wieder in die Tetraalkylester überführt werden. Die 1-Hydroxy-3-(N-methyl-N-propylamino)propan-1,1-diphosphonsäure der Formel I kann als freie Säure oder in Form ihres Mono-oder Dialkalisalzes isoliert werden. Die Alkalisalze lassen sich in der Regel durch Umfällen aus Wasser/Methanol oder Wasser/Aceton gut reinigen.

Als pharmakologisch verträgliche Salze werden vor allem Alkali- oder Ammoniumsalze verwendet, die man in üblicher Weise z.B. durch Titration der Verbindung mit anorganischen oder organischen Basen wie z.B. Natrium- oder Kaliumhydrogencarbonat, Natronlauge, Kalilauge, wässrigem Ammoniak oder Aminen wie z.B. Trimethyl- oder Triethylamin herstellt.

Die erfindungsgemäße neue Substanz der Formel I und ihre Salze können in flüssiger oder fester Form enteral oder parenteral appliziert werden. Hierbei kommen alle üblichen Applikationsformen in Frage, beispielsweise Tabletten, Kapseln, Dragees, Sirupe, Lösungen, Suspensionen etc.. Als Injektionsmedium kommt vorzugsweise Wasser zur Anwendung, welches die bei Injektionslösungen üblichen Zusätze wie Stabilisierungsmittel, Lösungvermittler und Puffer enthält. Derartige Zusätze sind z.B. Tartrat- und Citrat-Puffer, Ethanol, Komplexbildner (wie Ethylendiamintetraessigsäure und deren nichttoxische Salze), hochmolekulare Polymere (wie flüssiges Polyethylenoxid) zur Viskositätsregelung. Flüssige Trägerstoffe für Injektionslösungen müssen steril sein und werden vorzugsweise in Ampullen abgefüllt. Feste Trägerstoffe sind z.B. Stärke, Lactose, Mannit, Methylcellulose, Talkum , hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykole); für orale Applikation geeignete Zubereitungen können gewünschtenfalls Geschmacks- und Süßstoffe enthalten.

Die Dosierung kann von verschiedenen Faktoren, wie Applikationsweise, Spezies, Alter und/oder individuellem Zustand abhängen. Die täglich zu verabreichenden Dosen liegen bei etwa 1 - 1000 mg/Mensch, vorzugsweise bei 10 - 200 mg/Mensch und können auf einmal oder mehrere Male verteilt eingenommen werden.

Die nachfolgenden Beispiele zeigen einige der Verfahrensvarianten, die zur Synthese der erfindungsgemäßen Verbindung verwendet werden können. Sie sollen jedoch keine Einschränkung des Erfindungsgegenstandes darstellen. Die Struktur dieser Verbindung ist durch $^{1}H$- und $^{31}P$-NMR-Spektroskopie gesichert, die Reinheit mittels $^{31}P$-NMR-Spektroskopie, Dünnschichtelektrophorese (Cellulose, Oxalat-Puffer von pH = 4.0 ) und mittels C,H,N,P,Na-Analyse bestimmt. Zur Charakterisierung der Substanz wird der $M_{rel}$-Wert ( = relative Mobilität) bezogen auf Pyrophosphat ($M_{rel}$ = 1.0) angegeben.

Beispiel 1

1-Hydroxy-3-(N-methyl-N-propylamino)propan-1,1-diphosphonsäure

18 g 3-(N-Methyl-N-propylamino)-propionsäure werden mit 15 g phosphoriger Säure und 32 ml Phosphortrichlorid in 90 ml Chlorbenzol 12 Stunden bei 100 °C gehalten. Dann dekantiert man vom Lösungsmittel ab und rührt den Rückstand mit 250 ml 6 N Salzsäure 2 Stunden unter Rückfluß. Man filtriert von etwas Unlöslichem ab, konzentriert das Filtrat und gibt es auf eine Amberlite-Säule IR 120®, $H^+$-Form. Die Elution mit Wasser wird elektrophoretisch verfolgt. Die gewünschten Fraktionen werden vereinigt, eingeengt, mit Aceton/Methanol ausgerührt und die erhaltenen Kristalle isoliert.
Man erhält so 14.1 g Rohprodukt. Nach Umkristallisation aus Wasser/Methanol erhält man 9.8 g = 27 % analysenreines Produkt (x 1.5 $H_2O$), $M_{rel}$: 0.4, Fp. 96 - 102 °C (108 °C Zers.).

Die Ausgangsmaterial wird wie folgt erhalten:
N-Methyl-N-propylamin (JACS 79, 4720 (1957)) wird in Toluol mit Acrylsäure-methylester im Molverhältnis 1:3 umgesetzt und der mit 84 % Ausbeute erhaltene Ester ohne Destillation mit 1 N Natronlauge verseift. Man erhält so mit 92 % Ausbeute die ölige Säure, die ohne weitere Reinigung eingesetzt wird.

Beispiel 2

10 g 3-(N-Methyl-N-propylamino)propionsäure werden mit 11.4 g phosphoriger Säure 10 Minuten bei 80 °C zusammengeschmolzen. Man entfernt das Ölbad, tropft langsam 12 ml Phosphortrichlorid hinzu und erhitzt weitere 16 Stunden auf 80 °C.

Dann destilliert man das überschüssige Phosphortrichlorid ab, gibt 140 ml 6 N Salzsäure dazu und rührt 3 Stunden lang bei 100 °C. Man filtriert von etwas Unlöslichem ab, entfernt das Lösungsmittel im Vakuum und reinigt durch Ionenaustauscher-Chromatographie an Amberlite IR-120®
($H^+$-Form), wie in Bsp. 1 beschrieben. $M_{rel}$ = 0.4,
Ausbeute 6.95 g = 35 % (x 1.5 $H_2O$). Fp. 96 - 102 °C (108 °C Zers.).

Beispiel 3

15 g 3-(N-Methyl-N-propylamino)propionsäure werden analog zu Bsp. 2 mit 17 g $H_3PO_3$ erhitzt und mit 19 ml $POCl_3$ versetzt.

Nach 18 Stunden bei 80 °C wird das überschüssige $POCl_3$ abdestilliert und durch Zugabe von 210 ml 6 N Salzsäure und zweistündigem Erhitzen auf 100 °C hydrolysiert. Das Rohprodukt wurde (wie in Bsp. 1 beschrieben) durch Ionenaustauscher-Chromatographie an Amberlite IR-120®
($H^+$-Form) gereinigt. $M_{rel}$ = 0.4,
Ausbeute 16.2 g = 54 % nach Umkristallisation aus Wasser/Methanol Fp. 96 - 102 °C (108 °C Zers.).

Versuchsprotokoll

Männliche Wistar-Ratten mit einem Gewicht von ca. 160 g werden am Tag 1 thyroparathyroidektomisiert. Am Tag 5 wird der Erfolg der Operation dadurch kontrolliert, daß man nach einer Nacht Fasten die Calcaemie bestimmt. Von diesem Tag an erhalten alle Tiere dieselbe Menge Futter. Außerdem erhalten sie 3 Tage hintereinander subcutane Injektionen, wobei die eine 25 µg eines synthetischen Retinoids (zur Induzierung einer Hypercalcaemie), die andere das zu testende Diphosphonat enthält. Zusätzlich bekommen alle Tiere am ersten und letzten Tag der Behandlung 2 µg Thyroxin. 24 Stunden nach der letzten Retinoid- und Diphosphonat-Injektion und nach einer Nacht Fasten wird retroorbital unter Etheranesthesie Blut entnommen. Die Plasma-Calcium Konzentration wird durch Atomabsorption bestimmt.

Die Diphosphonate werden zuerst in einer Dosis von 0.1 mg P/kg in einem Volumen von 2 ml/kg, anschließend in einer Dosis von 0.01 und 0.001 mg P/kg verabreicht.

| | | mg P/kg | s.c. |
|---|---|---|---|
| | 0.001 | 0.01 | 0.1 |
| A | o | + | ++++ |
| B | | + | +++ |
| C | + | ++++ | ++++ |

| | | | | | |
|---|---|---|---|---|---|
| o | = Erniedrigung der Hypercalcaemie um | - 0.99 | bis | + 0.99 | mg % |
| (+) | = " " " | 1.0 | bis | 1.99 | " |
| + | = " " " | 2.0 | bis | 2.99 | " |
| ++ | = " " " | 3.0 | bis | 3.99 | " |
| +++ | = " " " | 4.0 | bis | 4.99 | " |
| ++++ | = " " " | >5.0 | | | " |

A = 1-Hydroxy-3-(N,N-dimethylamino)propan-1,1-diphosphonsäure (DE-OS 25 34 391)

B = 3-(N,N-Diethylamino)-1-hydroxypropan-1,1-diphosphonsäure (DE-OS 25 34 391)

C = 1-Hydroxy-3-(N-methyl-N-propylamino)propan-1,1-diphosphonsäure

**Patentansprüche**

**1.** 1-Hydroxy-3-(N-methyl-N-propylamino)propan-1,1-diphosphonsäure,

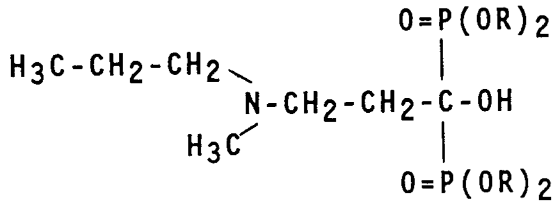

(I),

in der R Wasserstoff oder eine $C_1$ - $C_4$-Alkylgruppe bedeutet, sowie deren pharmakologisch unbedenkliche Salze.

**2.** Verfahren zur Herstellung von 1-Hydroxy-3-(N-methyl-N-propylamino)propan-1,1-diphosphonsäure, sowie deren Ester und deren pharmakologisch unbedenkliche Salze, dadurch gekennzeichnet, daß man

a) eine Carbonsäure der Formel II

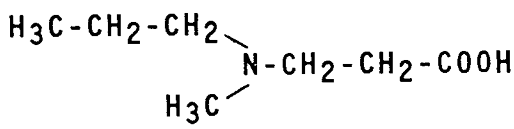

(II),

mit einem Gemisch aus phosphoriger Säure oder Phosphorsäure und einem Phosphorhalogenid oder Phosphorylhalogenid umsetzt und anschließend zur freien Diphosphonsäure der Formel I verseift,
oder
b) ein Carbonsäurechlorid der Formel III

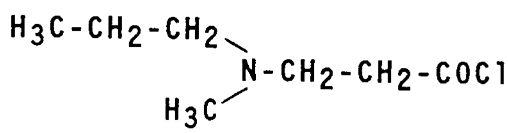

(III),

mit einem Trialkylphosphit der allgemeinen Formel IV

$P(OR')_3$ (IV),

in der R' für Alkylreste mit 1 - 4 Kohlenstoffatomen, vorzugsweise Methyl, Ethyl und Isobutyl steht, zu einem Acylphosphonat der Formel V

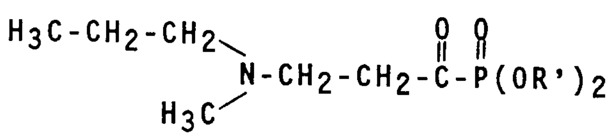

(V),

in der R' die oben angegebene Bedeutung hat,
umsetzt, anschließend mit einem Dialkylphosphit der allgemeinen Formel VI

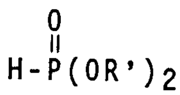

(VI),

in der R' die oben angegebene Bedeutung hat, zu einem Diphosphonat der Formel VII

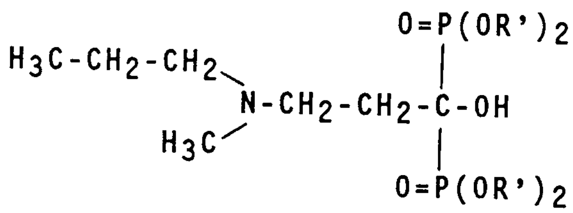

(VII),

in der R' die oben angegebene Bedeutung hat, reagieren läßt und gegebenenfalls die entstandenen Tetraester zu Diestern oder zur freien 1-Hydroxy-3-(N-methyl-N-propylamino)propan-1,1-diphosphonsäure der Formel I verseift,
oder
c) eine Verbindung der allgemeinen Formel VIII

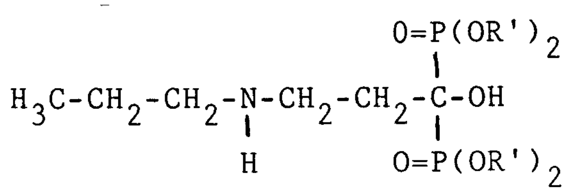

(VIII),

in der R' die oben angegebene Bedeutung hat, methyliert und gegebenenfalls die entstandenen Tetraester zu Diestern oder zur freien 1-Hydroxy-3-(N-methyl-N-propylamino)propan-1,1-diphosphonsäure der Formel I verseift, und die so hergestellten Verbindungen gegebenenfalls in ihre pharmakologisch verträglichen Salze überführt.

**3.** Arzneimittel enthaltend 1-Hydroxy-3-(N-methyl-N-propylamino)propan-1,1-diphosphonsäure oder deren Ester bzw. pharmakologisch unbedenkliche Salze gemäß Anspruch 1 sowie übliche pharmakologische Träger-und/oder Hilfsstoffe.

**4.** Verwendung von 1-Hydroxy-3-(N-methyl-N-propylamino)propan-1,1-diphosphonsäure sowie deren Ester und pharmakologisch unbedenkliche Salze gemäß Anspruch 1 zur Herstellung von Arzneimitteln.

**5.** "1-Hydroxy-3-(N-methyl-N-propylamino)propan-1,1-diphosphonsäure sowie deren Ester und pharmakologisch unbedenkliche Salze gemäß Anspruch 1 zur Behandlung von Calciumstoffwechselerkrankungen".

## Claims

**1.** 1-Hydroxy-3-(N-methyl-N-propylamino)-propane-1,1-diphosphonic acid,

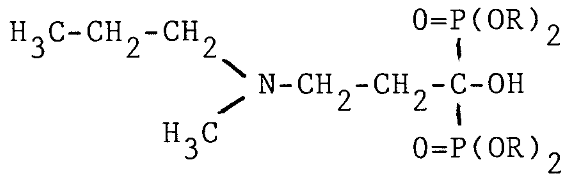

(I)

in which R signifies hydrogen or a $C_1$-$C_4$-alkyl group, as well as its pharmacologically acceptable salts.

**2.** Process for the preparation of 1-hydroxy-3-(N-methyl-N-propylamino)-propane-1,1-diphosphonic acid, as well as of its esters and its pharmacologically acceptable salts, characterised in that one

a) reacts a carboxylic acid of the formula II

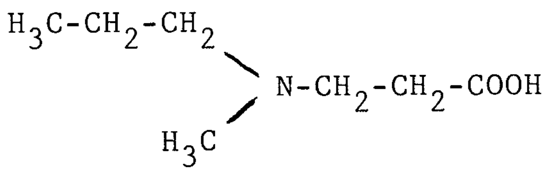

(II)

with a mixture of phosphorus acid or phosphoric acid and a phosphorus halide or phosphoryl halide and subsequently saponifies to the free diphosphonic acid of formula I, or

b) reacts a carboxylic acid chloride of the formula III

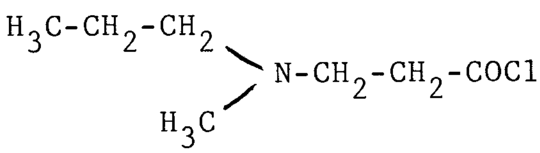

(III)

with a trialkyl phosphite of the general formula IV

$P(OR')_3$

in which R' stands for alkyl radicals with 1 - 4 carbon atoms, preferably methyl, ethyl and isobutyl, to an acyl phosphonate of the formula V

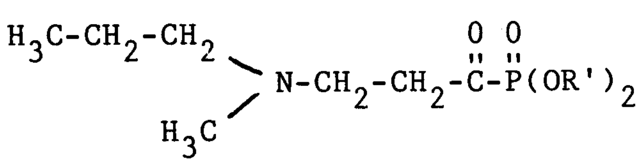

(V)

in which R' has the above-given meaning, subsequently allows to react with a dialkyl phosphite of the general formula VI

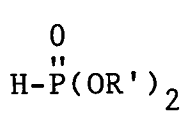

(VI)

in which R' has the above-given meaning, to a diphosphonate of the formula VII

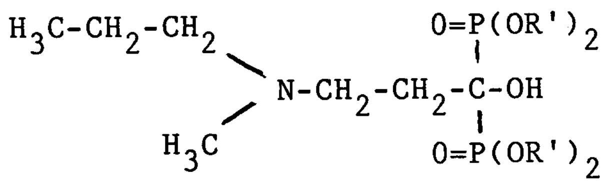

(VII)

in which R' has the above-given meaning, and possibly saponifies the resultant tetraesters to diesters or to the free 1-hydroxy-3-(N-methyl-N-propylamino)-propane-1,1-diphosphonic acid of the formula I, or

c) methylates a compound of the general formula VIII

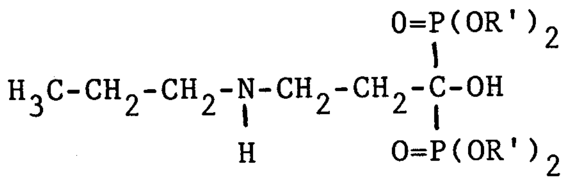

(VIII),

in which R' has the above-given meaning, and possibly saponifies the resultant tetraesters to diesters or to the free 1-hydroxy-3-(N-methyl-N-propylamino)-propane-1,1-diphosphonic acid of the formula I and possibly converts the so prepared compounds into their pharmacologically acceptable salts.

**3.** Medicaments containing 1-hydroxy-3-(N-methyl-N-propylamino)-propane-1,1-diphosphonic acid or its esters or pharmacologically acceptable salts according to claim 1, as well as usual pharmacological carrier and/or adjuvant materials.

**4.** Use of 1-hydroxy-3-(N-methyl-N-propylamino)-propane-1,1-diphosphonic acid, as well as of its esters and pharmacologically acceptable salts according to claim 1 for the preparation of medicaments.

**5.** 1-Hydroxy-3-(N-methyl-N-propylamino)-propane-1,1-diphosphonic acid, as well as its esters and pharmacologically acceptable salts according to claim 1 for the treatment of calcium metabolism diseases.

**Revendications**

**1.** Acides 1-hydroxy-3-(N-méthyl-N-propylamlino)propane-1,1-diphosphoniques,

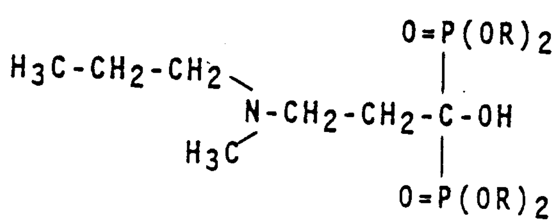

(I),

dans lesquels R représente un atome d'hydrogène ou un groupe alkyle en $C_1$-$C_4$, ainsi que leurs sels physiologiquement acceptables.

**2.** Procédé de fabrication d'acides 1-hydroxy-3-(N-méthyl-N-propylamino)propane-1,1-diphosphoniques, ainsi que de leurs esters et de leurs sels physiologiquement acceptables, caractérisé en ce que soit

a) on fait réagir un acide carboxylique de formule II

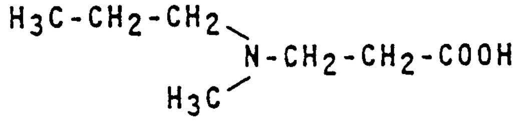

(II),

avec un mélange d'acide phosphoreux ou d'acide phosphorique et d'un halogénure de phosphore ou d'un halogénure de phosphoryle, et ensuite, on le saponifie en acide diphosphonique libre de formule I,
soit
b) on fait réagir un chlorure d'acide carboxylique de formule III

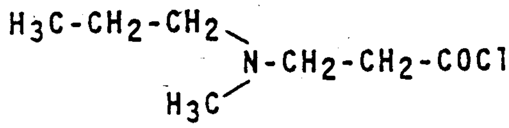

(III),

avec un phosphite de trialkyle de formule générale IV

$P(OR')_3$ (IV),

ou R' représente un groupe alkyle comportant 1-4 atomes de carbone, de préférence méthyle, éthyle et isobutyle, pour obtenir un phosphonate d'acyle de formule V

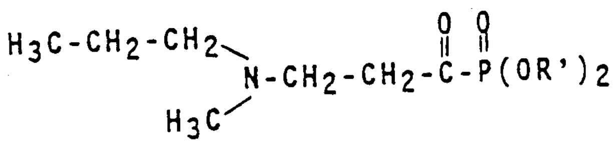

(V),

ou R' a la signification mentionnée plus haut, ensuite on fait réagir avec un phosphite de dialkyle de formule générale VI

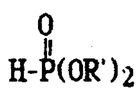

(VI),

ou R' a la signification mentionnée plus haut, pour obtenir un diphosphonate de formule VIII

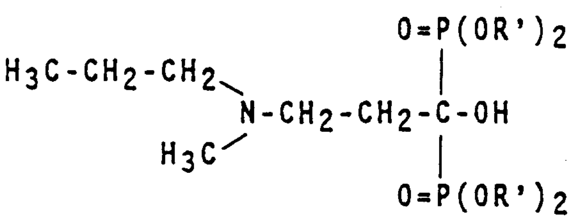

(VII),

où R' a la signification mentionnée plus haut, éveventuellement, on saponifie le tétraester obtenu en diester ou en acide 1-hydro-3-(N-méthyl-N-propylamino)propane-1,1-diphosphonique libre de formule I,
soit
c) on méthyle un composé de formule générale VIII

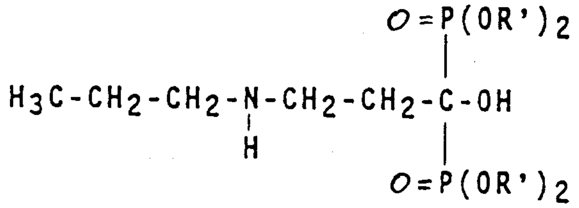

(VIII),

où R' a la signification mentionnée plus haut, éventuellement, on saponifie le tétraester obtenu en diester ou en acide 1-hydroxy-3-(N-méthyl-N-propylamino)propane-1,1-diphosphonique libre de formule I, et on transforme éventuellement le composé ainsi obtenu en un de ses sels pharmaceutiquement acceptables.

**3.** Produit pharmaceutique contenant des acides 1-hydro-3-(N-méthyl-N-propylamino)propane-1,1-diphosphoniques ou leurs esters ou sels pharmaceutiquement acceptables, selon la revendication 1, ainsi que des supports et/ou additifs pharmaceutiques usuels.

**4.** Utilisation des acides 1-hydro-3-(N-méthyl-N-propylamino)propane-1,1-diphosphoniques ou de leurs esters ou sels pharmaceutiquement acceptables, selon la revendication 1, pour la fabrication de produits pharmaceutiques.

**5.** Acides 1-hydro-3-(N-méthyl-N-propylamino)propane-1,1-diphosphoniques ainsi que leurs esters ou sels pharmaceutiquement acceptables selon la revendication 1, pour le traitement des troubles du métabolisme calcique.